(19) Europäisches Patentamt
European Patent Office
Office européen des brevets

(11) Numéro de publication: **0 177 390**
**A1**

(12) **DEMANDE DE BREVET EUROPEEN**

(21) Numéro de dépôt: 85401731.6

(22) Date de dépôt: 06.09.85

(51) Int. Cl.[4]: C 07 C 119/14
A 61 K 31/16, A 61 K 31/195
A 61 K 31/24

(30) Priorité: 27.09.84 FR 8414841

(43) Date de publication de la demande: 09.04.86 Bulletin 86/15

(84) Etats contractants désignés: AT BE CH DE FR GB IT LI LU NL SE

(71) Demandeur: SYNTHELABO
58, rue de la Glacière
F-75621 Paris Cedex 13(FR)

(72) Inventeur: Wick, Alexander
10, Boulevard des Plants
F-78860 St. Nom La Bretêche(FR)

(72) Inventeur: Raizon, Bernard
49, rue Gabriel Péri
F-91270 Vigneux(FR)

(74) Mandataire: Thouret-Lemaitre, Elisabeth et al.
Service Brevets - SYNTHELABO 58, rue de la Glacière
F-75621 Paris Cedex 13(FR)

(54) **Dérivés de diphénylazométhines à chaîne insaturée, leur préparation et leur application en thérapeutique.**

(57) Composés répondant à la formule générale I

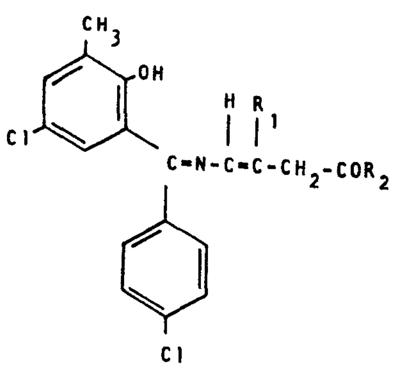

(I)

$R_1$ est un atome d'hydrogène ou le radical $CH_3$, et $R_2$ représente le groupe OH, OM (M = métal alcalin ou alcalinoterreux), $NH_2$, ou $O(C_{1-4}$ alkyle).

Application en thérapeutique.

EP 0 177 390 A1

La présente invention concerne des dérivés de diphénylazométhines à chaîne insaturée, leur préparation et leur application en thérapeutique.

Les composés de l'invention répondent à la formule (I) donnée dans l'annexe 1, dans laquelle
$R_1$ est un atome d'hydrogène ou le radical $CH_3$, et
$R_2$ représente le groupe OH, OM (M = métal alcalin ou alcalinoterreux), $NH_2$, ou $O(C_{1-4}$ alkyle).

Les composés de l'invention existent sous la forme d'isomères cis ou trans ou de mélanges de ces deux isomères.

La séparation en les deux isomères du mélange est effectuée par cristallisations successives.

Selon l'invention, on peut préparer les composés de l'invention selon le schéma réactionnel donné dans l'annexe 1.

On fait réagir la benzophénone (II) décrite par la demanderesse dans son brevet français 81 21 559 avec un composé (III) dans un solvant tel que le méthanol, à une température de 20°C à la température d'ébullition du solvant.

Les composés (III) sont préparés selon des méthodes classiques de la littérature à partir de composés connus.

Il est également possible de préparer les composés (I) dans lesquels $R_2$ est OM, $NH_2$ ou $O(C_{1-4}$ alkyle) à partir des composés (I) dans lesquels $R_2$ est OH, par salification, amidification ou estérification classique.

Les exemples suivants illustrent l'invention.
Les analyses et les spectres IR et RMN confirment la structure des composés.

<u>Exemple 1</u> Acide[[(chloro-5 hydroxy-2 méthyl-3 phényl) (chloro-4 phényl)méthylène]amino]-4 butène-3 oïque.

1. <u>Acide amino-4 butène-2 oïque</u>

On prépare ce composé à partir de l'acide D,L amino-4 hydroxy-3 butanoïque. On chauffe 15,5 g (0,13 mole) de cet acide dans 150 ml d'acide sulfurique concentré (36 N) à 128-130°C, pendant 1h. On dilue la solution obtenue à froid par 1000 ml d'eau. On ajoute alors 1200 g environ de Ba $CO_3$ jusqu'à pH 7,5. On agite longuement et vigoureusement la suspension. On filtre le précipité formé, le lave 3 fois avec de l'eau. On neutralise les ions $Ba^{++}$ avec de l'acide sulfurique N. On filtre la solution, la concentre à 50 ml, ajoute 150 ml d'éthanol et laisse cristalliser le composé. L'acide, mélange des deux isomères cis et trans, fond à 168-170°C en se décomposant.

2. <u>Acide[[(chloro-5 hydroxy-2 méthyl-3 phényl)(chloro-4 phényl)méthylène]amino]-4 butène-3 oïque</u>.

Dans un ballon de 1 l, on introduit 15,3 g (0,054 mole) de (chloro-5 hydroxy-2 méthyl-3 phényl)(chloro-4 phenyl)méthanone, 5 g (0,049 mole) d'acide amino-4 butène-2 oïque et 4,5g (0,054 mole) de carbonate acide de sodium, dans 700 ml de méthanol. On évapore le mélange à siccité, au bain d'eau à 100°C, sous pression réduite. On renouvelle sept fois l'opération en évaporant 500 ml de méthanol à la fois. On reprend le résidu sec par 200 ml de chlorure de méthylène et 300 ml d'eau, on acidifie à pH 4 avec 3,7 g d'acide citrique puis on extrait par 1 l de chlorure de méthylène. On lave la phase organique à l'eau, décante, sèche sur Mg $SO_4$, filtre et évapore à sec. On fait cristalliser le composé dans 100ml d'hexane, le filtre, l'essore et le dissout dans 100 ml d'acétate d'éthyle. Le produit cristallise lentement. On le filtre, l'essore et le lave avec 10 ml d'ether.

Après recristallisation, le composé qui est un mélange des deux isomères cis (45%) et trans (55%) fond à 146-7°C.

Exemple 2 Isomère trans de l'acide [[(chloro-5 hydroxy-2 méthyl-3 phényl)(chloro-4 phényl)méthylène] amino]-4 butène-3 oïque.

On isole l'isomère trans par recristallisations successives dans de l'acétate d'éthyle.
Le composé fond à 202-203°C.

Exemple 3 [[(chloro-5 hydroxy-2 méthyl-3 phényl)(chloro-4 phényl)méthylène]amino]-4 butène-3 amide et son isomère trans.

A 3,3 g de l'acide obtenu dans l'exemple 1, et dissous dans 100 ml de tetrahydrofuranne (THF), on ajoute 1,6 g de carbonyldiimidazole et on agite le mélange pendant 2 h. On prépare une solution d'ammoniac gazeux dans 250 ml de THF anhydre dans laquelle on introduit lentement la première solution obtenue. On agite le mélange pendant 3 h et laisse reposer pendant la nuit. On évapore à sec sous pression réduite et partage le résidu entre 150ml de $CH_2Cl_2$ et 100ml d'eau. On décante la phase organique, sèche sur Mg $SO_4$, filtre et évapore à sec. On obtient une huile que l'on purifie par chromatographie sur silice (éluant : acétate d'éthyle). Les 3 dernières fractions contenant les produits sont séchées sur Mg $SO_4$. On filtre et évapore à sec les filtrats. Parmi les 3 résidus obtenus, on fait recristalliser l'un des résidus, mélange des 2 isomères cis (32%) / trans (68%) dans un mélange d'éther et d'éther de pétrole.
F = 170-171°C.

L'isomère trans est obtenu par recristallisation de l'un des résidus dans un mélange éther / éther de pétrole. Il fond à 191-192°C.

Exemple 4 [[(chloro-5 hydroxy-2 méthyl-3 phényl)(chloro-4 phényl)méthylène]amino]-4(méthyl-3 butène-3 oate) d'éthyle.

1. Amino-4 méthyl-3 butène-2 oate d'éthyle.

1.1. A 13 g de méthyl-3 butène-2 oate d'éthyle dissous dans 130 ml de $CCl_4$, on ajoute 20 g de N-bromo-succinimide recristallisé et une pointe de spatule de peroxyde de benzoyle. On chauffe le mélange à la température du reflux, en l'agitant, pendant 3 h. Après refroidissement, on filtre le précipité et le rince avec 20 ml de $CCl_4$. On concentre le filtrat et distille sous pression réduite.
$Eb_5$ = 75°C

$n_D^{26}$ = 1,4932.

1.2. On agite en chauffant une solution de 5,3 g d'hexaméthylènetétramine dans 45 ml de $CHCl_3$ et on ajoute lentement 7 g du composé bromé obtenu sous 1.1. On chauffe le mélange à la température du reflux en agitant pendant 3 h. On le laisse reposer pendant la nuit. Après avoir refroidi au bain d'eau glacée, on filtre le précipité que l'on essore et sèche à l'étuve. On dissout 5,2 g de ce produit intermédiaire, composé ammonium quaternaire, dans 10 ml d'eau, 50ml d'éthanol et 12 ml d'acide chlorydrique concentré. On laisse reposer le mélange pendant 24 h. On filtre et concentre à sec. On reprend le résidu dans de l'alcool, filtre le précipité de chlorure d'ammonium et évapore à sec le filtrat. On obtient le composé, sous forme de chlorhydrate, solide huileux utilisé tel quel dans l'étape suivante.

2. [[(chloro-5 hydroxy-2 méthyl-3 phényl)(chloro-4 phényl)méthylène] amino]-4(méthyl-3 butène-3 oate) d'éthyle.

A 6 g du chlorhydrate obtenu sous 1.2., dissous dans 150 ml de méthanol, on ajoute 5,6 g de bicarbonate de sodium. On évapore à sec la solution sous vide, à la température ambiante. On ajoute alors 400 ml de méthanol et 9 g de

(chloro-5 hydroxy-2 méthyl-3 phényl)(chloro-4 phényl)méthanone. On évapore le mélange à sec au bain-marie à 80°C. On met le mélange sous vide. Puis on évapore trois fois de suite 400 ml de méthanol dans les mêmes conditions. On partage le résidu entre 200 ml d'éther et 250 ml d'eau, décante, sèche sur $MgSO_4$, filtre et évapore à sec. On sépare les composés par chromatographie sur silice en éluant au chlorure de méthylène. On fait recristalliser l'un des résidus dans du pentane. On obtient l'isomère trans.

F = 81-82°C.

On fait recristalliser un autre résidu dans du pentane avec traitement au charbon végétal. On obtient l'isomère cis.

F = 96-97°C.

Les composés de l'invention préparés à titre d'exemples sont rassemblés dans le tableau suivant :

<u>Tableau</u>

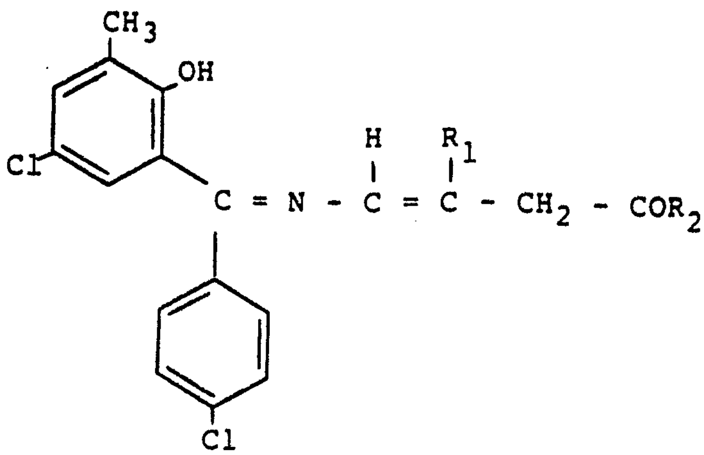

(I)

| Composé | $CH=\underset{R_1}{C}-CH_2$ | $R_2$ | mélange (m) ou isomère | F (°C) |
|---|---|---|---|---|
| 1 | $-CH=CH-CH_2-$ | OH | m | 146-7 |
| 2 | $-CH=CH-CH_2-$ | OH | trans | 202-3 |
| 3 | $-CH=CH-CH_2-$ | $OCH_3$ | m | 85-6 |
| 4 | $-CH=CH-CH_2-$ | $NH_2$ | trans | 191-2 |
| 5 | $-CH=CH-CH_2-$ | $NH_2$ | m | 170-1 |
| 6 | $-CH=CH-CH_2-$ | ONa | m | 215-6 |
| 7 | $-CH=\underset{CH_3}{C}-CH_2-$ | $OC_2H_5$ | cis | 96-7 |
| 8 | $-CH=\underset{CH_3}{C}-CH_2-$ | $OC_2H_5$ | trans | 81-2 |

Les composés de l'invention ont été soumis à des essais pharmacologiques montrant leur activité sur le système nerveux central.
La toxicité aigüe a été déterminée chez la souris par voie intrapéritonéale. La DL 50 (dose léthale 50%) induisant la mort chez 50% des animaux va de 500 à > 1000 mg/kg.

L'activité antidépressive des composés a été montrée par l'antagonisme vis à vis des "head-twitches" (ébrouements de la tête) provoqués par le L-5-hydroxy-tryptophane chez la souris.

Les souris (mâles CD1, Charles River France ; 18-22 g de poids corporel) reçoivent les produits à étudier, à doses croissantes, ou le solvant, simultanément avec le L-5-HTP à la dose de 250 mg/kg, par voie sous-cutanée. Quarante cinq minutes après cette injection de 5-HTP, le nombre de "head-twitches" (ébrouements de la tête) est compté, pour chaque souris, pendant une minute.

Pour chaque traitement, la moyenne des "head-twitches", ainsi que le pourcentage de variation par rapport au lot témoin, sont calculés.

A partir de la courbe effet-dose, on détermine la DA 50 (dose active 50% ou dose qui diminue de 50% le nombre moyen de "head-twitches"), par la méthode graphique de Miller et Tainter (1944).

La DA 50 des composés de l'invention varie de 20 à 60 mg/kg par voie orale.

L'activité anticonvulsivante des composés a été montrée par l'antagonisme vis à vis de la mortalité induite par la bicuculline chez la souris.

La bicuculline est un bloqueur relativement sélectif des récepteurs GABA-ergiques post synaptiques et ses effets convulsivants et létaux sont antagonisés par les composés élevant le taux de GABA cérébral ou possédant une activité GABA-mimétique.

On a évalué la dose active 50% (DA 50), dose protégeant 50% des animaux contre l'effet de la bicuculline, des substances étudiées.

La DA 50 des composés de l'invention varie de 20 à 100 mg/kg par voie orale.

Les composés de l'invention sont actifs comme antidépresseurs et anticonvulsivants et possèdent également des propriétés anxiolytiques, analgésiques et antiinflammatoires. Ils sont utilisables en thérapeutique humaine et vétérinaire pour le traitement de diverses maladies du système nerveux central, par exemple pour le traitement des dépressions, des psychoses, de certaines maladies neurologiques comme l'épilepsie, la spasticité, la diskynésie.

L'invention comprend, par conséquent, toutes compositions pharmaceutiques renfermant les composés (I) comme principes actifs, en association avec tous excipients appropriés à leur administration, en particulier par voie orale (comprimés, dragées, gélules, capsules, cachets, solution ou suspensions buvables) ou parentérale.

La posologie quotidienne peut aller de 100 à 3000 mg.

Annexe 1



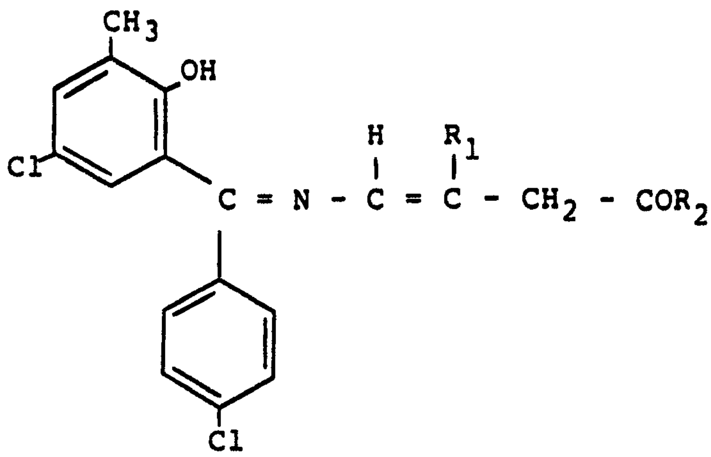

(I)

Schéma réactionnel

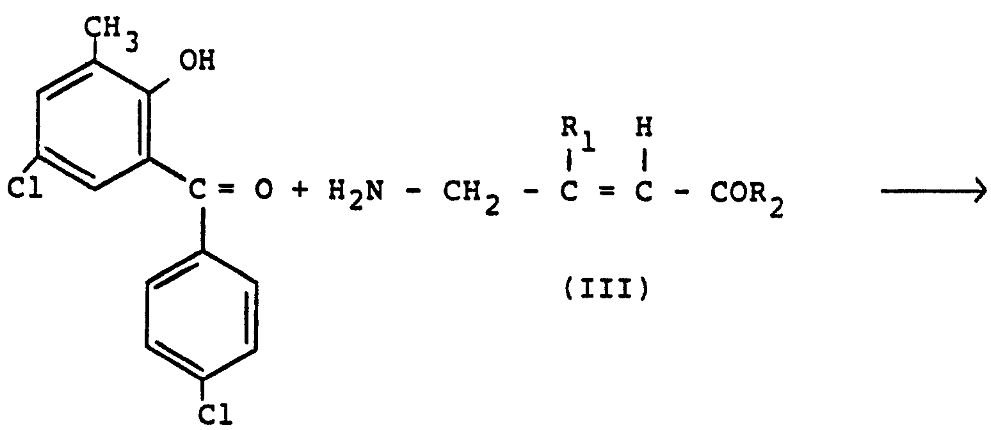

(III)

(I)

(II)

Revendications pour les états contractants : BE, CH, DE, FR, GB, IT, LI, LU, NL, SE.

1. Dérivés de diphénylazométhines à chaîne insaturée, sous la forme d'isomères cis ou trans ou de mélanges de ces deux isomères, répondant à la formule (I)

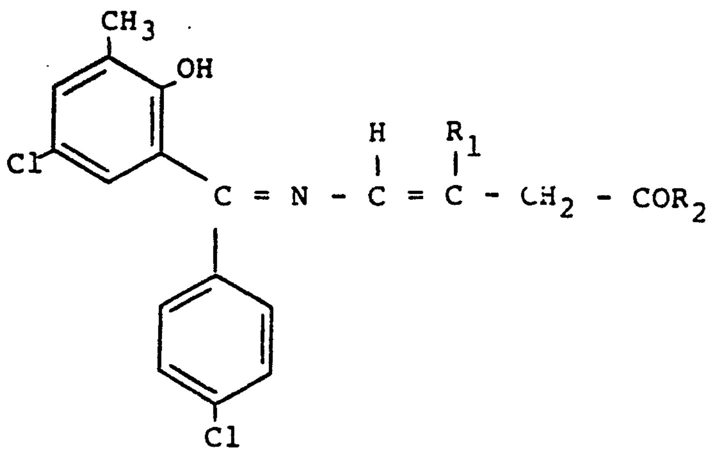

(I)

$R_1$ est un atome d'hydrogène ou le radical $CH_3$, et $R_2$ représente le groupe OH, OM (M = métal alcalin ou alcaninoterreux), $NH_2$, ou $O(C_{1-4}$ alkyle).

2. Procédé de préparation des composés spécifiés dans la revendication 1, procédé caractérisé en ce que l'on fait réagir une benzophénone de formule (II)

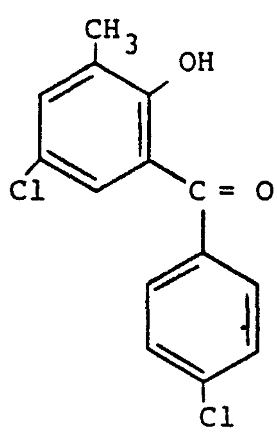

(II)

avec un composé (III)

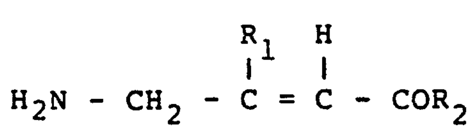

(III)

3. Médicament caractérisé en ce qu'il contient un composé spécifié dans la revendication 1.

4. Composition pharmaceutique caractérisée en ce qu'elle contient un composé spécifié dans la revendication 1 en association avec tout excipient approprié.

Revendication pour l'état contractant : AT.

Procédé de préparation de dérivés de diphénylazométhines à chaîne insaturée, sous la forme d'isomères cis ou trans ou de mélanges de ces deux isomères, répondant à la formule (I)

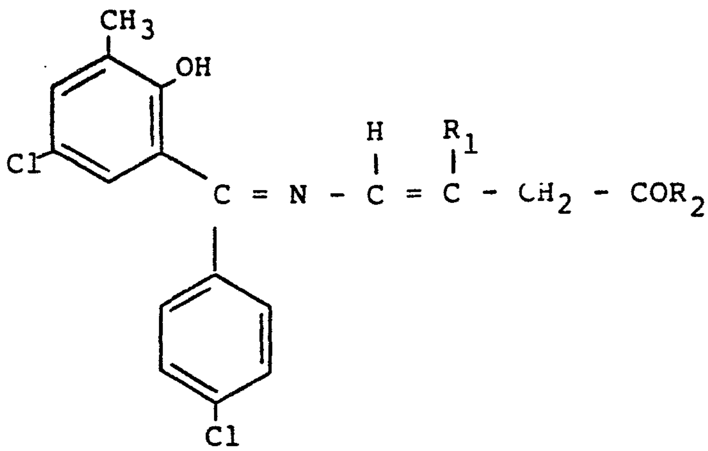

(I)

dans laquelle
$R_1$ est un atome d'hydrogène ou le radical $CH_3$, et
$R_2$ représente le groupe OH, OM (M = métal alcalin ou alcaninoterreux), $NH_2$, ou $O(C_{1-4}$ alkyle),
procédé caractérisé en ce que l'on fait réagir une benzophénone de formule (II)

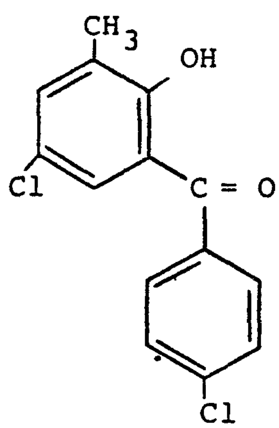

(II)

avec un composé (III)

$$H_2N - CH_2 - \overset{R_1}{\overset{|}{C}} = \overset{H}{\overset{|}{C}} - COR_2 \qquad (III),$$

les radicaux ayant les définitions données ci-dessus.

European Patent Office

# EUROPEAN SEARCH REPORT

0177390
Application number

EP 85 40 1731

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 516 509 (SYNTHELABO)<br>* Revendications *<br>----- | 1-4 | C 07 C 119/14<br>A 61 K 31/16<br>A 61 K 31/195<br>A 61 K 31/24 |
| | | | **TECHNICAL FIELDS SEARCHED (Int. Cl.4)** |
| | | | C 07 C 119/20 |

The present search report has been drawn up for all claims

| Place of search | Date of completion of the search | Examiner |
|---|---|---|
| THE HAGUE | 19-12-1985 | GAUTIER R.H.A. |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document